# EUROPEAN PATENT SPECIFICATION

(11) **EP 1 800 591 B1**
(45) Date of publication and mention of the grant of the patent: **06.06.2012**
(21) Application number: 04792496.4
(22) Date of filing: 15.10.2004
(51) Int. Cl.: A61B 1/00, A61B 1/04

(54) **RADIO INTRA-SUBJECT INFORMATION ACQUIRING SYSTEM**
RADIO-INFORMATIONSERFASSUNGSSYSTEM IN EINEM PROBANDEN
SYSTEME RADIO D'ACQUISITION D'INFORMATIONS INTRA-SUJET

(43) Date of publication of application: 27.06.2007
(73) Proprietor: Olympus Corporation, Tokyo 151-0072 (JP)
(72) Inventor: SHIMIZU, Hatsuo c/o Olympus Corporation, Tokyo 151-0072 (JP); NAKATSUCHI, Kazutaka c/o Olympus Med. Systems Corp., Tokyo 151-0072 (JP)
(74) Representative: von Hellfeld, Axel
(86) International application number: PCT/JP2004/015279
(87) International publication number: WO 2006/040830

(56) References cited:
- JP-A- 11 205 056
- JP-A- 2001 231 186
- JP-A- 2001 231 186
- JP-A- 2003 135 389
- US-A- 5 807 258
- US-A1- 2003 114 742
- US-A1- 2003 139 661
- US-A1- 2003 171 653
- US-A1- 2004 152 988

## Description

### TECHNICAL FIELD

The present invention relates to a radio intra-subject information acquiring system that includes a body-insertable device which is inserted into a subject and acquires information of the subject, and a receiving device which is disposed at the outside of the subject and receives intra-subject information radio-transmitted from the body-insertable device.

### BACKGROUND ART

In recent years, a swallowable capsule endoscope has emerged in the field of endoscope. The capsule endoscope is provided with an imaging function and a radio communication function. The capsule endoscope has such a function that after the capsule endoscope is swallowed from the mouth of a subject for the purpose of observation (examination), the capsule endoscope moves inside body cavities, for example, internal organs such as a stomach and a small intestine following peristaltic motions of the organs, and sequentially picks up images, until the capsule endoscope is naturally discharged.

In a period during which the capsule endoscope moves inside the body cavity, image data that is picked up inside the body by the capsule endoscope is sequentially transmitted to the outside by radio communication, and is stored in a memory provided at the outside. By carrying a receiver having a radio communication function and a memory function, the subject can freely move after swallowing the capsule endoscope until the capsule endoscope is discharged. After the capsule endoscope is discharged, a doctor or a nurse can diagnose by displaying the images of the organ on a display, based on the image data stored in the memory.

While the capsule endoscope may have a configuration in which driving power is obtained from an incorporated power source, a configuration which recently attracts attention supplies driving power to the capsule endoscope by radio transmission through a transmission antenna provided at the outside. When the power is supplied from the outside, it is possible to prevent an unintended power exhaustion from stopping the driving of the capsule endoscope in the middle of the movement of the capsule endoscope inside the body cavity (see, for example, Patent Document 1).

Patent Document 1: Japanese Patent Application Laid-open No. 2001-231186 (Page 3, and FIG. 1)
US 2003/0139661 A1 relates to an ingestible device adapted to travel in the gastrointestinal tract and performs a diagnostic image of tissues therein.
US 2004/0152988 A1 relates to a capsule imaging system which provides the function of examining the gastro-intestinal system and nearby tissues using ultra-wideband imaging technology.
US 2003/0114742 A1 provides a system and method for controlling a device in vivo.
JP 2001-231186 A relates to a power transmission system for operating an apparatus, e. g. a radio capsule, held in a living body for a long time.
US 2003/0171653 A1 relates to a capsule endoscope provided with lighting means for illuminating the interior of a living body, an image pickup means for capturing an image of a site illuminated by the lighting means, and a transparent cover which houses the image pickup means and the lighting means within a sealed, cylindrical-shaped capsule case.

### DISCLOSURE OF INVENTION

### PROBLEM TO BE SOLVED BY THE INVENTION

However, a conventional capsule endoscope system has a problem in that it is difficult to obtain a clear image of the body cavity. This problem is explained in detail below.

The capsule endoscope system transfers image data by radio transmission, unlike an ordinary image acquiring apparatus such as a digital camera. Therefore, there is a problem that a noise signal is easily mixed into the image data before the signal is processed, unlike data transmitted by a cable connection. Particularly, because the capsule endoscope is inserted into the subject, a transmission antenna and the like need to be incorporated in a considerably small capsule. Therefore, transmission strength is lower than transmission strength achieved by a radio transmission mechanism employed in other fields.

On the other hand, in the capsule endoscope system, it is highly necessary to acquire image data which is clear enough to allow for image recognition. This is because the capsule endoscope system is mainly used for medical diagnosis and the like.

It is necessary to acquire as clearer image data as possible partly because of characteristics of the operation of the capsule endoscope itself. Specifically, the moving speed of the capsule endoscope within the body cavity has position dependency, and the capsule endoscope moves at a considerably fast speed within the esophagus, for example. When the capsule endoscope picks up images within the body cavity approximately every 0.5 second, for example, the number of image data acquired in an area such as the esophagus where the capsule endoscope moves at a fast speed is smaller than the number of image data acquired in other areas. Therefore, to make the diagnosis or the like based on the image data acquired through imaging in the area where the capsule endoscope moves fast, it is highly necessary that the acquired image data include as few unclear image data as possible.

As explained above, according to the conventional capsule endoscope system, it is difficult to acquire a satisfactory image, despite high necessity for acquiring clearer image data. This problem is also present when certain intra-subject information, other than the image data acquired by the capsule endoscope, acquired by the body-insertable device is transferred via a radio transmission.

The present invention is achieved in view of the above. It is an object of the present invention to securely acquire intra-subject information such as image data at the outside, by suppressing a mixing of a noise signal, for example, in an intra-subject information acquiring system that transfers information from a body-insertable device such as a capsule endoscope to the outside via a radio transmission.

### MEANS FOR SOLVING PROBLEM

In order to achieve the object by solving the above problems, a radio intra-subject information acquiring system according to claim 1 is provided.

In the radio intra-subject information acquiring system, an amplifier is disposed near a receiving antenna. Therefore, it is possible to suppress attenuation of a radio signal from a body-insertable device received by the receiving antenna, and a mixing of a noise signal, whereby intra-subject information can be securely acquired in the receiving device.

In the intra-subject information acquiring system, the receiving device further includes a receiving jacket having a shape that the subject can wear, and the receiving antenna unit and the amplifier are disposed on the receiving jacket.

In the intra-subject information acquiring system, plural receiving antenna units are disposed, and plural amplifiers are disposed near the receiving antenna units, respectively.

In the intra-subject information acquiring system, the receiving device further includes a power transmitter that transmits power to the body-insertable device, and a power recovering unit that recovers at least a part of power transmitted from the power transmitter, and the amplifiers operate using power recovered by the power recovering unit as a driving source.

In the intra-subject information acquiring system, a power recovering coil may be disposed to cover a receiving antenna unit and the like. Therefore, a noise signal generated at the outside can be electrostatically shielded, whereby intra-subject information can be acquired even more securely.

### EFFECT OF THE INVENTION

The radio intra-subject information acquiring system according to the present invention has the amplifier disposed near the receiving antenna unit. Therefore, there is an effect that it is possible to suppress attenuation of a radio signal from the body-insertable device received by the receiving antenna unit, and a mixing of a noise signal, whereby intra-subject information can be securely acquired in the receiving device.

The radio intra-subject information acquiring system according to the present invention has the power recovering coil formed to cover the receiving antenna unit and the like. Therefore, there is an effect that a noise signal generated at the outside can be electrostatically shielded, whereby intra-subject information can be acquired even more securely.

### BRIEF DESCRIPTION OF DRAWINGS

FIG. 1 is a schematic diagram showing an overall configuration of a radio intra-subject information acquiring system according to a first embodiment;
FIG. 2 is a block diagram schematically showing a configuration of a capsule endoscope that constitutes the radio intra-subject information acquiring system;
FIG. 3 is a block diagram schematically showing a configuration of a receiving device that constitutes the radio intra-subject information acquiring system;
FIG. 4 is a schematic diagram showing one example of a disposition of receiving antennas and amplifiers;
FIG. 5 is a schematic diagram showing another example of a disposition of receiving antennas and amplifiers;
FIG. 6 is a schematic diagram showing another example of a disposition of receiving antennas and amplifiers;
FIG. 7 is a block diagram schematically showing a configuration of a transmitting/receiving jacket that constitutes a radio intra-subject information acquiring system according to a second embodiment; and
FIG. 8 is a schematic diagram for explaining a positional relationship and the like between antennas that are disposed within a transmitting/receiving jacket.

### EXPLANATIONS OF LETTERS OR NUMERALS

- 1: Subject
- 2: Capsule endoscope
- 3: Receiving device
- 3a: External device
- 3b: Transmitting/receiving jacket
- 4: Display device
- 5: Portable recording medium
- 11: LED
- 12: LED driving circuit
- 13: CCD
- 14: CCD driving circuit
- 15: RF transmitting unit
- 16: Transmitting antenna unit
- 17: Receiving antenna unit
- 19: Separating circuit
- 20: Power reproducing circuit
- 21: Booster circuit
- 22: Capacitor
- 23: Control-information detecting circuit
- 24: System control circuit
- 25: RF receiving unit
- 26: Image processing unit
- 27: Storage unit
- 28: Oscillator
- 29: Control-information input unit
- 30: Superimposing circuit
- 31: Amplifier circuit
- 32: Power supply unit
- 41: Transmitting/receiving jacket
- 42: Power recovering antenna
- 42a: Power recovering coil
- 43: Separating circuit
- 44: Power reproducing circuit
- 45: Booster circuit
- 46: Capacitor
- A1 to An: Receiving antenna
- B1 to Bm: Power feeding antenna
- C1 to Cn: Amplifier
- D1 to Dn: Conducting wire

### BEST MODE(S) FOR CARRYING OUT THE INVENTION

A radio intra-subject information acquiring system will be explained below as best modes for carrying out the invention. Note that the drawings are schematic, and that a relationship between a thickness and a width of each part, and a rate of a thickness of each part are different from actual data. Needless to mention, a size relationship and rates are different between the drawings. In the following embodiments, explanations are given based on a capsule endoscope system that images a body cavity, as an example. However, intra-subject information is not limited to a body cavity image, and it is needless to mention that the radio intra-subject information acquiring system is not limited to a capsule endoscope system.

### First embodiment

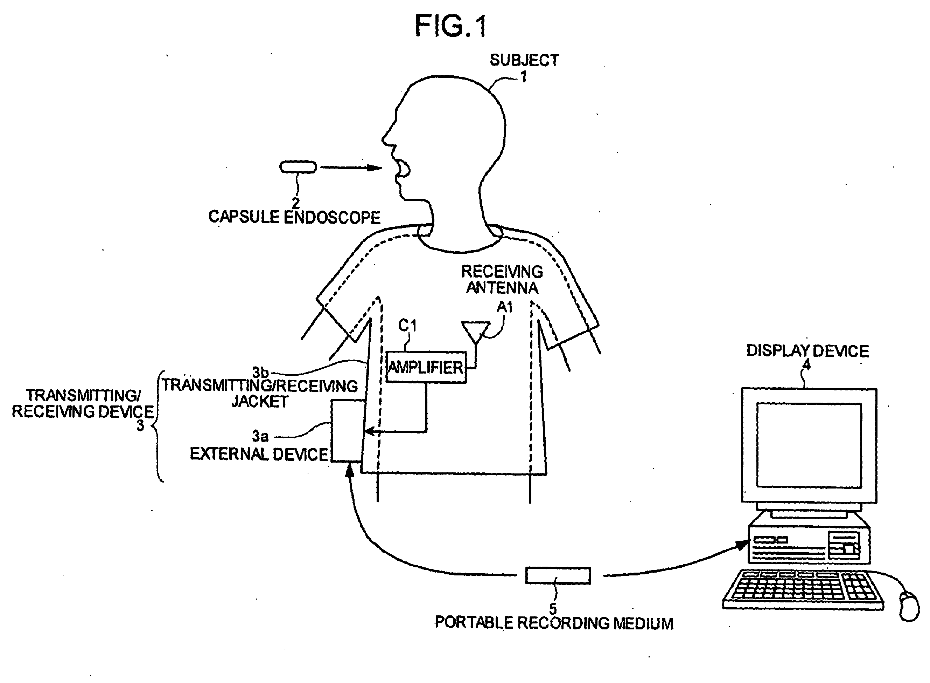

FIG. 1 is a schematic diagram showing an overall configuration of a radio intra-subject information acquiring system according to a first embodiment. As shown in FIG. 1, the radio intra-subject information acquiring system includes a capsule endoscope 2 that is inserted into the body of a subject 1, and images the body cavity, and a receiving device 3 that has a function of radio transmission and reception to and from the capsule endoscope 2. The radio intra-subject information acquiring system includes a display device 4 that displays a body-cavity image based on data received by the receiving device 3, and a portable recording medium 5 that delivers data between the receiving device 3 and the display device 4.
The receiving device 3 includes a transmitting/receiving jacket 3b worn by the subject 1, and an external device 3a that performs processing of radio signals, for example, transmitted and received via the transmitting/receiving jacket 3b. A receiving antenna A1 and an amplifier C1 are disposed in the transmitting/receiving jacket 3b.

The display device 4 displays body-cavity images picked up by the capsule endoscope 2, and has a configuration such as a workstation that displays images based on data acquired by the portable recording medium 5. Specifically, the display device 4 can have a configuration that directly displays images with a CRT display, a liquid display, or the like, or the display device 4 can have a configuration that outputs images to other medium as in a printer.

The portable recording medium 5 has a configuration that is attachable/detachable to/from the external device 3a and the display device 4, and can output or record information like a Compact Flash (registered trademark) memory, when the portable recording medium 5 is mounted to the external device 3a and the display device 4. Specifically, the portable recording medium 5 records data transmitted from the capsule endoscope 2 by being mounted to the external device 3a while the capsule endoscope 2 is moving within the body cavity of the subject 1. After the capsule endoscope 2 is discharged to the outside from the subject 1, the portable recording medium 5 is taken out from the external device 3a, and is inserted into the display device 4. The display device 4 reads the recorded data. Because the portable recording medium 5 delivers data between the external device 3a and the display device 4, the subject 1 can freely behave while the body cavity is being imaged, unlike when the external device 3a and the display device 4 are connected together by wire.

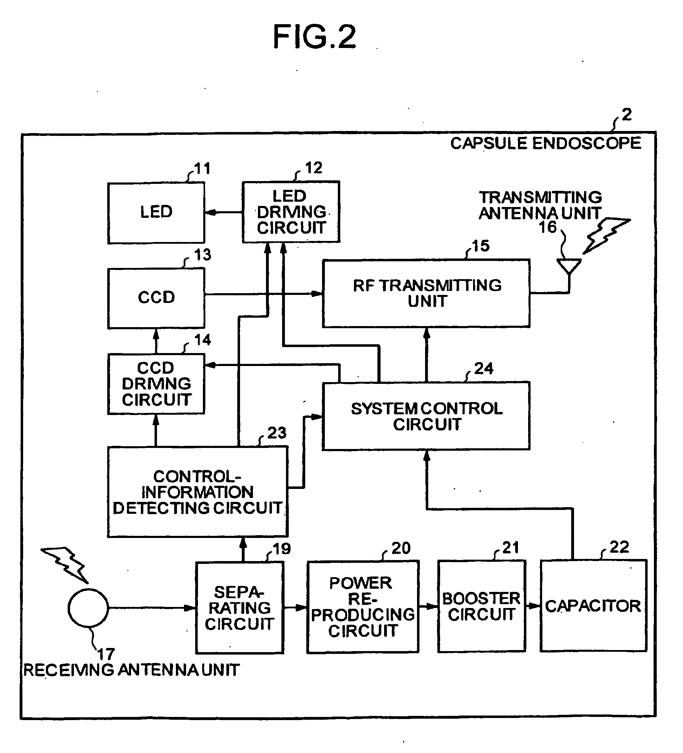

The capsule endoscope 2 is inserted into the subject 1, images the body cavity, and transmits the acquired image data to the receiving device 3. FIG. 2 is a schematic diagram showing a configuration of the capsule endoscope 2. As shown in FIG. 2, the capsule endoscope 2 includes an LED 11 that irradiates an imaging area at the time of imaging of the inside of the subject 1, an LED driving circuit 12 that controls a driving state of the LED 11, and a CCD 13 that images the area irradiated by the LED 11. The capsule endoscope 2 includes a CCD driving circuit 14 that controls a driving state of the CCD 13, an RF transmitting unit 15 that generates an RF signal by modulating the image data imaged by the CCD 13, a transmitting antenna unit 16 that transmits the RF signal output from the RF transmitting unit 15, and a system control circuit 24 that controls an operation of the LED driving circuit 12, the CCD driving circuit 14, and the RF transmitting unit 15.

Being provided with these mechanisms, the capsule endoscope 2 acquires image information of an examined area of the subject irradiated by the LED 11 with the CCD 13, while the capsule endoscope 2 is in the subject 1. The RF transmitting unit 15 converts the acquired image information into an RF signal, and the RF signal is transmitted to the outside via the transmitting antenna unit 16.

The capsule endoscope 2 also includes a receiving antenna unit 17 that receives a radio signal transmitted from the receiving device 3, and a separating circuit 19 that separates a power feeding signal from a signal received by the receiving antenna unit 17. Further, the capsule endoscope 2 includes a power reproducing circuit 20 that reproduces power from the separated power feeding signal, a booster circuit 21 that boosts the reproduced power, and a capacitor 22 that stores the boosted power. Further, the capsule endoscope 2 includes a control-information detecting circuit 23 that detects the content of a control information signal from a component separated from the power feeding signal by the separating circuit 19, and outputs a control signal to the LED driving circuit 12, the CCD driving circuit 14, and the system control circuit 24, as necessary.

In the capsule endoscope 2 provided with these mechanisms, the receiving antenna unit 17 first receives a radio signal transmitted from the receiving device 3, and the separating circuit 19 separates a power feeding signal and a control information signal from the radio signal. The control information signal is output to the LED driving circuit 12, the CCD driving circuit 14, and the system control circuit 24, via the control-information detecting circuit 23, and is used to control the driving states of the LED 11, the CCD 13, and the RF transmitting unit 15. On the other hand, the power reproducing circuit 20 reproduces power from the power feeding signal. The booster circuit 21 boosts the reproduced power to a potential of the capacitor 22, and the boosted power is stored in the capacitor 22. The capacitor 22 supplies power to the system control circuit 24 and other constituent elements. In this way, the capsule endoscope 2 has a configuration that receives power based on a radio transmission from the receiving device 3.

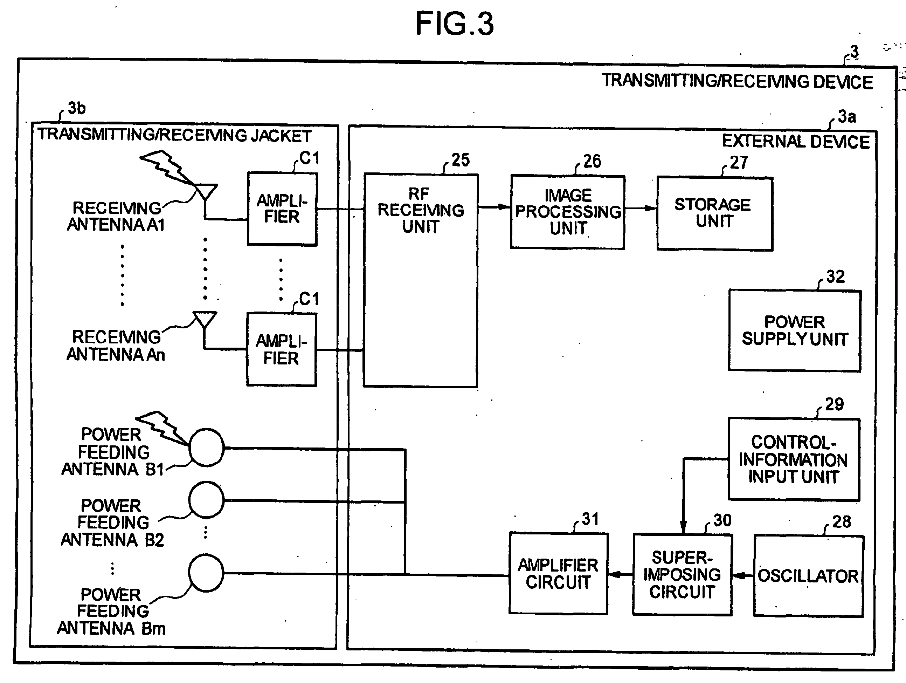

The receiving device 3 is explained next. The receiving device 3 receives, via a radio transmission, image data acquired by the capsule endoscope 2, and transmits a radio signal including a power feeding signal to be converted into power, to the capsule endoscope 2. FIG. 3 is a block diagram schematically showing a configuration of the receiving device 3. The configuration of the receiving device is explained below with reference to FIG. 3.

The receiving device 3 includes the external device 3a and the transmitting/receiving jacket 3b as shown in FIG. 1. As shown in FIG. 3, the external device 3a includes an RF receiving unit 25 that performs predetermined process to a radio signal transmitted from the capsule endoscope 2, extracts image data acquired by the capsule endoscope 2 from the radio signal, and outputs the extracted image data, an image processing unit 26 that performs necessary process to the output image data, and a storage unit 27 that stores the image-processed image data. Image data is recorded into the portable recording medium 5 via the storage unit 27.

The external device 3a has a function of generating a radio signal to be transmitted to the capsule endoscope 2. Specifically, the external device 3a includes an oscillator 28 that generates a power feeding signal and prescribes an oscillation frequency, a control-information input unit 29 that generates a control information signal to control a driving state of the capsule endoscope 2, a superimposing circuit 30 that combines the power feeding signal with the control information signal, and an amplifier circuit 31 that amplifies strength of the combined signal. A signal amplified by the amplifier circuit 31 is transmitted to power feeding antennas B1 to Bm described later, and is transmitted to the capsule endoscope 2. The external device 3a also includes a power supply unit 32 having a predetermined capacitor or an AC power source adapter. Constituent elements of the external device 3a and amplifiers C1 to Cn described later use power supplied from the power supply unit 32 as driving energy.

The transmitting/receiving jacket 3b has a shape wearable by the subject 1, and can perform radio communication with the capsule endoscope 2 when the transmitting/receiving jacket 3b is worn after the capsule endoscope 2 is inserted into the subject 1. Specifically, the transmitting/receiving jacket 3b includes the power feeding antennas B1 to Bm that transmit a radio signal including a power feeding signal to the capsule endoscope 2. The power feeding antennas B1 to Bm are connected to the amplifier circuit 31 provided in the external device 3a, and can transmit a radio signal generated by the external device 3a.

The transmitting/receiving jacket 3b also includes the receiving antennas A1 to An that receive radio signals transmitted from the capsule endoscope 2, and the amplifiers C1 to Cn that are disposed near the receiving antennas A1 to An, and amplify the radio signals received by the receiving antennas A1 to An. Each of the amplifiers C1 to Cn is connected to the RF receiving unit 25 provided in the external device 3a. The radio signals amplified by the amplifiers C1 to Cn are output to the RF receiving unit 25, and are subjected to a predetermined process. The amplifiers C1 to Cn can use configurations of ordinary amplifier circuits, and can have not only a function of amplifying strength but also a function of rejecting a signal component having a frequency other than a specific frequency band.

The term "near" to the receiving antennas A1 to An means that a length of a conducting wire that connects the receiving antennas A1 to An and the amplifiers C1 to Cn, respectively is such a distance that the attenuation amount of a radio signal is suppressed to a permissible range and that the mixing amount of a noise signal is suppressed to a permissible range. Specifically, when the receiving antennas A1 to An are disposed within the transmitting/receiving jacket 3b as in the first embodiment, the term "near" means a state that the amplifiers C1 to Cn are disposed within the transmitting/receiving jacket 3b. More preferably, a distance between each of the amplifiers C1 to Cn and the corresponding one of the receiving antennas A1 to An is set shorter than a distance between two receiving antennas among the receiving antennas A1 to An.

In the radio intra-subject information acquiring system according to the first embodiment, the amplifiers C1 to Cn are disposed near the receiving antennas A1 to An, within the transmitting/receiving jacket 3b. Based on this configuration, when the receiving device 3 receives image data acquired by the capsule endoscope 2 via a radio communication, the radio intra-subject information acquiring system suppresses attenuation of the received radio signal and occurrence of degradation in the image data due to the mixing of a noise signal. These advantages are explained below.

According to the conventional radio intra-subject information acquiring system, the amplifier that amplifies a radio signal received by the receiving antenna is usually disposed within the external device 3a, to avoid complexity of the configuration and to suppress power loss. However, when the conventional configuration is employed, there are problems that the radio signal received by the receiving antenna is attenuated by a large amount before the radio signal reaches the amplifier to be amplified, and that a noise signal is mixed into the radio signal. As a result, the external device 3a cannot easily restore the image data acquired by the capsule endoscope, and there is a possibility that the display device 4 has a difficulty in displaying a clear image after the image data is transferred to the display device 4 via the receiving device 3.

Therefore, in the first embodiment, the amplifiers C1 to Cn are disposed near the receiving antennas A1 to An so that the receiving device 3 can restore high-precision image data, and acquire clear image data. Specifically, according to the first embodiment, the amplifiers C1 to Cn are disposed near the corresponding receiving antennas A1 to An, respectively. With this arrangement, the radio signals received by the receiving antennas A1 to An can be amplified before being attenuated. When the amplifiers C1 to Cn are disposed near the receiving antennas A1 to An, a mixture of a noise signal in the signals amplified by the amplifiers C1 to Cn can be decreased. Therefore, the amplifiers C1 to Cn can suppress amplification gain to some extent, and can avoid amplification of a noise signal when performing signal amplification. Accordingly, the image data transferred from the capsule endoscope 2 via a radio transmission can be restored in high precision, and the display device 4 can display a clear image.

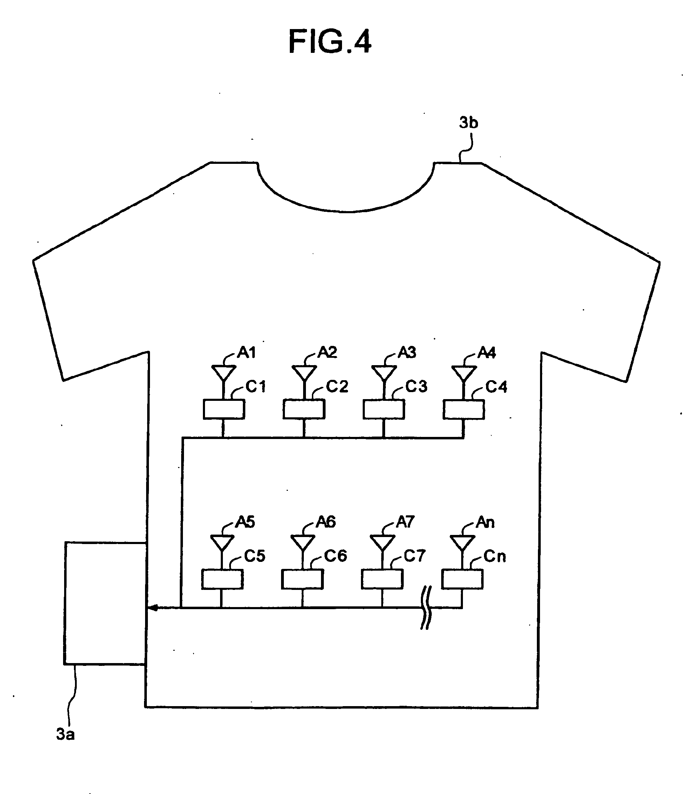

The receiving antennas and the amplifiers can be disposed in various ways so long as the condition of mutual proximity is satisfied. For example, as shown in FIG. 4, receiving antennas and amplifiers can be provided in one-to-one relationship. In the configuration shown in FIG. 4, when plural receiving antennas are disposed, amplifiers can be disposed near the corresponding receiving antennas.
Therefore, particularly clear images can be acquired.

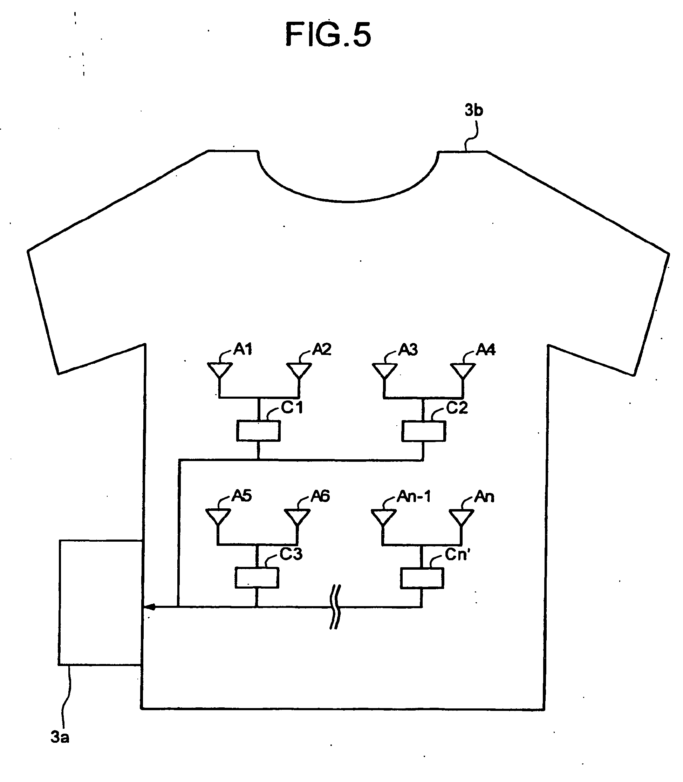

A configuration as shown in FIG. 5 is also effective as other example of disposition. In the example shown in FIG. 5, among plural receiving antennas disposed, those receiving antennas adjacent to each other are grouped, and an amplifier is disposed for each group. Thus, the amplifiers can be disposed near the receiving antennas.

In the example shown in FIG. 5, the configuration is particularly effective when the receiving antennas are suitably switched over during their use. Depending on the configuration of the radio intra-subject information acquiring system, it is effective to have such a configuration that only a receiving antenna positioned nearest to the capsule endoscope 2 is used as a receiving antenna according to the position of the capsule endoscope 2, which is a body-insertable device, in the body cavity, and that the receiving antenna is suitably switched to the other receiving antenna along with the move of the capsule endoscope 2. Then, a radio signal acquired from a receiving antenna which is not currently functioning as a receiving antenna does not need to be amplified. Therefore, it is effective to dispose the amplifiers such that the amplifiers amplify only the radio signals acquired by receiving antennas that function as receiving antennas simultaneously.

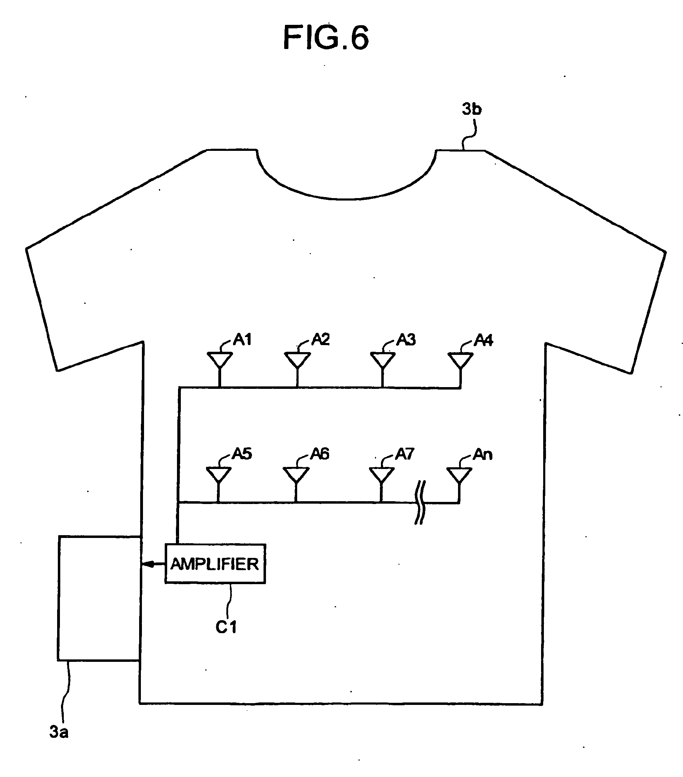

Further, as a simple configuration, an example shown in FIG. 6 is also effective. The configuration shown in FIG. 6 has an advantage in that only one amplifier is sufficient, although a distance between receiving antennas and the amplifier is larger than that in the configurations shown in FIGS. 4 and 5. In the configuration shown in FIG. 6, the amplifier is provided within at least the transmitting/receiving jacket 3b. Therefore, the amplifier is disposed near the receiving antennas, and a clear image can be acquired.

### Second embodiment

A radio intra-subject information acquiring system according to a second embodiment is explained next. The radio intra-subject information acquiring system according to the second embodiment further includes a power recovering antenna that is disposed on the transmitting/receiving jacket, and recovers a part of radio signals transmitted from the receiving device, and has a configuration that power acquired by the power recovering antenna is used as driving power of the amplifier. In the second embodiment, the capsule endoscope 2, the display device 4, and the portable recording medium 5 have configurations similar to those of the first embodiment.

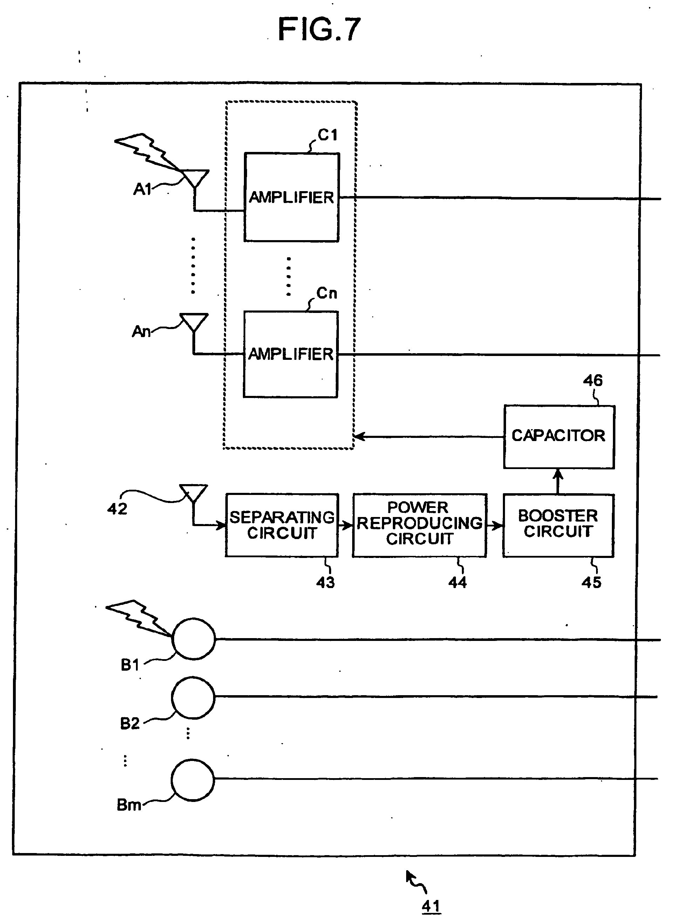

FIG. 7 is a block diagram that shows a configuration of a transmitting/receiving jacket 41 which is part of the receiving device according to the second embodiment. The transmitting/receiving jacket 41 includes the receiving antennas A1 to An, the power feeding antennas B1 to Bm, and the amplifiers C1 to Cn that are similar to those of the first embodiment. The transmitting/receiving jacket 41 also includes a power recovering antenna 42 that receives a part of radio signals that are not received by the capsule endoscope 2, among radio signals transmitted from the power feeding antennas B1 to Bm, a separating circuit 43 that reproduces power based on the radio signal received by the power recovering antenna 42, a power reproducing circuit 44, a booster circuit 45, and a capacitor 46.

The separating circuit 43, the power reproducing circuit 44, the booster circuit 45, and the capacitor 46 have functions similar to the functions of the separating circuit 19, the power reproducing circuit 20, the booster circuit 21, and the capacitor 22 shown in FIG. 2. The power recovering antenna 42 can take an optional configuration so as to be able to receive a radio signal including a power feeding signal. It is assumed here that the power recovering antenna 42 is formed with a power recovering coil.

Power recovery in the second embodiment is briefly explained. As already explained, the external device 3a has a configuration that radio transmits a power feeding signal to be converted into driving power within the capsule endoscope 2, together with control information, via the power feeding antennas B1 to Bm. However, because the capsule endoscope 2 is small, and because a moving range of the capsule endoscope 2 is wide, the capsule endoscope 2 fails to receive a large part of the transmitted radio signals, and power transmission efficiency is poor. Therefore, in the second embodiment, a power recovering coil 42a receives radio signals that are not received by the capsule endoscope 2. The separating circuit 43 is used to reproduce power, and the reproduced power is used as driving power of the amplifiers C1 to Cn. Thus, it is not necessary to provide a power supply line from the external device 3a to the amplifiers C1 to Cn, whereby the load on the subject 1 who wears the transmitting/receiving jacket 41 is decreased.

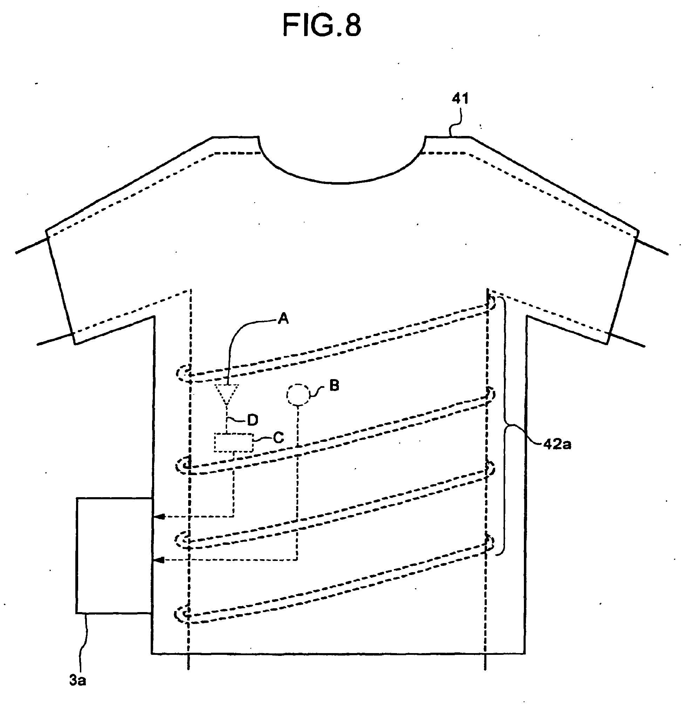

FIG. 8 is a schematic diagram for explaining a relationship between antennas disposed within the transmitting/receiving jacket 41. As shown in FIG. 8, within the transmitting/receiving jacket 41, there are disposed a receiving antenna A, an amplifier C, a conducting wire D that electrically connects the receiving antenna A and the amplifier C, and a power feeding antenna B. The power recovering coil 42a that forms the power recovering antenna 42 is formed to cover the above elements. Specifically, in the second embodiment, the power recovering coil 42a is configured to surround the subject 1, the power feeding antennas B1 to Bm, the receiving antennas A1 to An, the amplifiers C1 to Cn, and conducting wires D1 to Dn, when the subject 1 wears the transmitting/receiving jacket 41.

Advantages of the radio intra-subject information acquiring system according to the second embodiment are explained next. First, in the second embodiment, since the power recovering antenna 42, the separating circuit 43, the power reproducing circuit 44, the booster circuit 45, and the capacitor 46 are provided, the radio intra-subject information acquiring system can effectively use radio signals that are transmitted from the power feeding antennas B1 to Bm and not received by the capsule endoscope 2. While it is difficult to directly improve power transmission efficiency, a part of the transmitted radio signals are received and are used again as power of the receiving device. Therefore, power efficiency can be improved as a whole, and power consumption can be decreased. Particularly, in the second embodiment, as shown in FIG. 8, because the power recovering coil 42a is disposed so as to surround the power feeding antennas B1 to Bm, the power recovering coil 42a can efficiently receive radio signals transmitted from the power feeding antennas B1 to Bm, whereby power consumption is further decreased.

Further, in the second embodiment, because the mechanism that reproduces power is disposed within the transmitting/receiving jacket 41, loss of power supplied from the capacitor 46 to the amplifiers C1 to Cn can be decreased. Since there is a certain distance between the external device and the amplifiers C1 to Cn provided within the transmitting/receiving jacket 41, if the amplifiers C1 to Cn receive power from the capacitor within the external device, a part of the power is converted into heat or the like before reaching the amplifiers C1 to Cn, and the power supply cannot be efficiently performed. On the other hand, according to the second embodiment, the capacitor 46 is disposed within the transmitting/receiving jacket 41, and the capacitor 46 can be disposed near the amplifiers C1 to Cn. Therefore, power can be supplied efficiently. Particularly, the power recovering antennas 42, the separating circuit 43, the power reproducing circuit 44, and the booster circuit 45 are disposed within the transmitting/receiving jacket 41 to recover power stored in the capacitor 46. Therefore, there is no problem in disposing the capacitor 46 in the transmitting/receiving jacket 41. On the contrary, it is advantageous to dispose the capacitor 46 within the transmitting/receiving jacket 41, because the recovered power can be stored in the capacitor 46 more efficiently. In the second embodiment, since the power supply unit 32 shown in FIG. 3 supplies power to the constituent elements of the external device, the disposition of the capacitor 46 in the transmitting/receiving jacket 41 does not make power supply to the external device less efficient.

Further, in the second embodiment, disposition of the power recovering coil 42a that constitutes the power recovering antenna 42 has an advantage in that degradation of image data received by the receiving antennas A1 to An can be suppressed and that clear image data can be acquired. As shown in FIG. 8, the power recovering coil 42a is formed to surround the receiving antennas A1 to An, the amplifiers C1 to Cn, and the conducting wires D1 to Dn that connect the receiving antennas A1 to An and the amplifiers C1 to Cn, when the subject 1 wears the transmitting/receiving jacket 41. In the configuration shown in FIG. 8, the power recovering coil 42a not only functions as a part of the antenna, but also can exhibit the function of electrically shielding the receiving antennas A1 to An from radio signals generated in the surrounding environment. Therefore, it is possible to suppress the mixing of a noise signal generated at the outside of the transmitting/receiving jacket 41 into the radio signal via the receiving antennas A1 to An and the conducting wires D1 to Dn. The amplifiers C1 to Cn amplify the radio signals, into which the mixing of a noise signal is suppressed. As a result, degradation of the acquired image data can be suppressed, and a clear image can be acquired.

In other words, in the second embodiment, the power recovering coil 42a has not only the function of re-receiving the radio signals transmitted via the power feeding antenna B1 to Bm but also the electric shielding function, whereby the power supply efficiency can be improved and the degradation of image data can be suppressed. This shielding function exerts the effect particularly in hospital rooms in which various diagnostic apparatuses are disposed. Specifically, a mixing of a noise signal into the radio signals received by the transmitting/receiving jacket 41, attributable to electromagnetic waves generated from the diagnostic apparatuses can be substantially suppressed. Because the power feeding antennas B1 to Bm as well as the receiving antennas A1 to An are disposed within the power recovering coil 42a, radio signals transmitted by the power feeding antennas B1 to Bm are naturally present within the power recovering coil 42a. However, when the frequency bands of the transmitted radio signals are set to bands that cannot be received by the receiving antennas A1 to An, the radio signals transmitted from the power feeding antenna B1 to Bm do not function as noise signals in the receiving antennas A1 to An.

While the present invention has been explained above with reference to the first embodiment and the second embodiment, the present invention is not limited to the above, and those skilled in the art can reach various embodiments, modifications, and application examples. For example, in the first and the second embodiments, the capsule endoscope includes an LED, a CCD, and the like, thereby picking up images inside the subject 1. However, a body-insertable device that is inserted in the subject is not limited to have the above configuration, and can acquire other intra-subject information such as temperature information and pH information. The body-insertable device may include an oscillator to acquire ultrasonic images in the subject 1. The oscillator can acquire plural pieces of information from the intra-subject information.

In the first embodiment, a radio signal transmitted via the power feeding antennas B1 to Bm can be either of the power feeding signal and the control information signal. Alternatively, other different signal can be superimposed on the radio signal, and the superimposed signal can be transmitted. Alternatively, the power feeding antennas B1 to Bm can be omitted, and the receiving device 3 can have only the function of receiving and processing the radio signal transmitted from the capsule endoscope 2. In other words, the present invention can be applied to any system as far as the system includes at least a mechanism of transferring intra-subject information from the capsule endoscope 2 to the outside via radio transmission.

In the second embodiment, the capacitor 46 can be omitted. While the capsule endoscope 2 moves within the subject 1, the power feeding antennas B1 to Bm constantly transmit radio signals each including a power feeding signal. Because the power recovering antenna 42 is disposed in a predetermined area within the transmitting/receiving jacket 41, the power recovering antenna 42 continues recovering at least a part of the radio signals transmitted from the power feeding antennas B1 to Bm constantly, if a suitable adjustment is made at the initial setting time. Therefore, the power recovering antenna 42 can continue recovering the radio signal without stopping a recovery operation, until the capsule endoscope 2 is discharged to the outside of the body. Power can be steadily supplied to the amplifiers C1 to Cn, so that a stable operation can be performed even when the capacitor 46 is omitted.

In the second embodiment, instead of the arrangement that only the capacitor 46 supplies driving power to the amplifiers C1 to Cn, the power supply unit that is provided within the external device can supply a part of the driving power. This configuration has an advantage in that the power supply efficiency is improved, as compared with the power supply efficiency when the power recovering antenna 42 is not provided. In addition, the power recovering antenna 42 can continue exerting the electrostatic shielding function.

While the display device 4 displays only the input image data in the above, a predetermined image process, for example, can be performed as necessary. The display device 4 can have a large-capacity storage device to record image data, and can reference image data that is picked up in the past.

### INDUSTRIAL APPLICABILITY

As explained above, the radio intra-subject information acquiring system according to the present invention is useful for a swallowable capsule endoscope that is used in the medical field, for example. Particularly, the radio intra-subject information acquiring system is suitable for a system that suppresses a mixing of a noise signal into a radio signal transmitted from a body-insertable device such as a capsule endoscope and that securely acquires intra-subject information such as image data at the outside.

## Claims

1. A radio intra-subject information acquiring system including a body-insertable device (2) that is insertable into a subject (1) and adapted to acquire information of the subject (1), and a receiving device (3) that is configured to be disposed outside the subject (1) and adapted to receive intra-subject information which is radio transmitted from the body-insertable device (2), wherein
the receiving device (3) includes
a receiving jacket having a shape that the subject can wear,
receiving antenna units (A1 to An) that are disposed within the receiving jacket and adapted to receive a radio signal transmitted from the body-insertable device (2),
a plurality of amplifiers (C1 to Cn) that are disposed within the receiving jacket and adapted to amplify the radio signal while avoiding a mixing of a noise signal into the radio signal, and
a signal processor (25) that is adapted to process the radio signal amplified by the amplifiers (C1 to Cn),
**characterized in that**
a distance between each of the amplifiers (C1 to Cn) and a corresponding one of the receiving antenna units (A1 to An) is set shorter than a distance between two receiving antenna units among the receiving antenna units (A1 to An), and
the length of a conducting wire that connects the receiving antenna units (A1 to An) and the amplifiers (C1 to Cn), respectively, is such a distance that the attenuation amount of the radio signal is suppressed to a permissible range and that the mixing amount of the noise signal is suppressed to a permissible range, wherein
the receiving device (3) further includes:
a power transmitter (B1 to Bm) adapted to transmit power to the body-insertable device (2), and
a power recovering unit (42) that is disposed on the receiving jacket and adapted to recover at least a part of power transmitted from the power transmitter (B1 to Bm), and wherein
the amplifiers (C1 to Cn) are adapted to operate using power recovered by the power recovering unit (42) as a driving source.

2. The radio intra-subject information acquiring system according to claim 1, wherein
the power recovering unit (42) includes a power recovering coil (42a), and
the power recovering coil (42a) is disposed to cover the receiving antenna units (A1 to An) and a wiring configuration that connects the receiving antenna units (A1 to An) and the amplifiers (C1 to Cn).

## Patentansprüche

1. Funksystem zum Erfassen von patienten-interner Information, mit einer in einen Körper einführbaren Vorrichtung (2), die in einen Patienten (1) einführbar ist und Information über den Patienten (1) zu erfassen vermag, und einer Empfangsvorrichtung (3), die konfiguriert ist, um außerhalb des Patienten (1) angeordnet zu werden, und die patienten-interne Information, die per Funk von der in den Körper einführbaren Vorrichtung (2) übertragen wird, zu empfangen vermag, wobei
die Empfangsvorrichtung (3) umfasst
eine Empfangsjacke mit einer Form, die der Patient tragen kann, Empfangsantenneneinheiten (A1 bis An), die in der Empfangsjacke angeordnet sind und ein von der in den Körper einführbaren Vorrichtung (2) übertragenes Funksignal zu empfangen vermögen,
mehrere Verstärker (C1 bis Cn), die in der Empfangsjacke angeordnet sind und das Funksignal zu verstärken vermögen, während sie ein Vermischen eines Rauschsignals mit dem Funksignal verhindern, und
einen Signalprozessor (25), der das von den Verstärkern (C1 bis Cn) verstärkte Funksignal zu verarbeiten vermag,
**dadurch gekennzeichnet, dass**
ein Abstand zwischen jedem der Verstärker (C1 bis Cn) und einer entsprechenden der Empfangsantenneneinheiten (A1 bis An) kürzer eingestellt ist als ein Abstand zwischen zwei Empfangsantenneneinheiten unter den Empfangsantenneneinheiten (A1 bis An), und
die Länge eines Leitungsdrahts, der jeweils die Empfangsantenneneinheiten (A1 bis An) und die Verstärker (C1 bis Cn) verbindet, so lang ist, dass der Dämpfungsbetrag des Funksignals auf einen zulässigen Bereich gemindert wird und der Mischbetrag des Rauschsignals auf einen zulässigen Bereich gemindert wird, wobei
die Empfangsvorrichtung (3) ferner umfasst:
einen Energieüberträger (B1 bis Bm), der auf die in den Körper einführbare Vorrichtung (2) Energie zu übertragen vermag, und
eine Energie-Rückgewinnungseinheit (42), die an der Empfangsjacke angeordnet ist und zumindest einen Teil der von dem Energieüberträger (B1 bis Bm) übertragenen Energie zurückzugewinnen vermag, und wobei
die Verstärker (C1 bis Cn) unter Nutzung der von der Energie-Rückgewinnungseinheit (42) zurückgewonnenen Energie als Antriebsquelle zu arbeiten vermögen.

2. Funksystem zum Erfassen von patienten-interner Information nach Anspruch 1, wobei
die Energie-Rückgewinnungseinheit (42) eine Energie-Rückgewinnungsspule (42a) aufweist, und
die Energie-Rückgewinnungsspule (42a) so angeordnet ist, dass sie die Empfangsantenneneinheiten (A1 bis An) und eine Verdrahtungskonfiguration, welche die Empfangsantenneneinheiten (A1 bis An) mit den Verstärkern (C1 bis Cn) verbindet, bedeckt.

## Revendications

1. Système radio d'acquisition d'informations intra-sujet comprenant un dispositif pouvant être inséré dans un corps (2) qui peut être inséré dans un sujet (1) et adapté pour acquérir des informations du sujet (1), et un dispositif de réception (3) qui est configuré pour être disposé à l'extérieur du sujet (1) et adapté pour recevoir des informations intra-sujet qui sont télétransmises depuis le dispositif pouvant être inséré dans un corps (2), dans lequel
le dispositif de réception (3) comprend
une chemise de réception présentant une forme que le sujet peut porter, des unités d'antennes de réception (A1 à An) qui sont disposées à l'intérieur de la chemise de réception et adaptées pour recevoir un signal radio transmis depuis le dispositif pouvant être inséré dans un corps (2),
une pluralité d'amplificateurs (C1 à Cn) qui sont disposés à l'intérieur de la chemise de réception et adaptés pour amplifier le signal radio tout en évitant un mélange d'un signal bruit dans le signal radio, et
un processeur de signal (25) qui est adapté pour traiter le signal radio amplifié par les amplificateurs (C1 à Cn),
**caractérisé en ce que**
une distance entre chacun des amplificateurs (C1 à Cn) et l'une correspondante parmi les unités d'antennes de réception (A1 à An) est établie plus courte qu'une distance entre deux unités d'antennes de réception parmi les unités d'antennes de réception (A1 à An), et
la longueur d'un fil conducteur qui connecte les unités d'antennes de réception (A1 à An) et les amplificateurs (C1 à Cn), respectivement, est d'une distance telle que la quantité d'atténuation du signal radio est amenée à une plage admissible et que la quantité de mélange du signal bruit est amenée à une plage admissible, dans lequel
le dispositif de réception (3) comprend en outre :
un transmetteur de puissance (B1 à Bm) adapté pour transmettre la puissance vers le dispositif pouvant être inséré dans un corps (2), et
une unité de récupération de puissance (42) qui est disposée sur la chemise de réception et adaptée pour récupérer au moins une partie de la puissance transmise depuis le transmetteur de puissance (B1 à Bm), et dans lequel
les amplificateurs (C1 à Cn) sont adaptés pour fonctionner en utilisant la puissance récupérée par l'unité de récupération de puissance (42) en tant que source d'entraînement.

2. Système radio d'acquisition d'informations intra-sujet selon la revendication 1, dans lequel
l'unité de récupération de puissance (42) comprend une bobine de récupération de puissance (42a), et
la bobine de récupération de puissance (42a) est disposée pour couvrir les unités d'antennes de réception (A1 à An) et une configuration de câblage qui connecte les unités d'antennes de réception (A1 à An) et les amplificateurs (C1 à Cn).
